Europäisches Patentamt

European Patent Office

Office européen des brevets

(19)

(11) Publication number: **0 277 925**
**A1**

# EUROPEAN PATENT APPLICATION

(12)

(21) Application number: 88850006.3

(22) Date of filing: 13.01.88

(51) Int. Cl.⁴: **A 61 K 31/635**
**A 61 K 9/54**

(30) Priority: 15.01.87 SE 8700137

(43) Date of publication of application:
10.08.88 Bulletin 88/32

(84) Designated Contracting States:
AT BE CH DE ES FR GB IT LI LU NL SE

(71) Applicant: LEJUS MEDICAL AKTIEBOLAG
Taljegardsgatan 3
S-431 33 Mölndal (SE)

(72) Inventor: Appelgren, Curt Henry
Benjaminssons väg PI 1461
S-434 00 Kungsbacka (SE)

Eskilsson, Eva Christina
Körsbärsvägen 15
S-435 00 Mölnlycke (SE)

(74) Representative: Inger, Lars Ulf Bosson
L + U INGER Patentbyra HB Garvaregatan 12
S-262 00 Ängelholm (SE)

(54) Diuretic composition.

(57) The present invention relates to a multiple-unit dose composition comprising furosemide as pharmaceutically active compound, whereby the composition gives a release of furosemide in accordance with Dissotest in a buffer having pH 1.5 of at most 4% during hour 1, a release in a buffer having pH 5.5 of at most 7.5% during a consequtive hour (hour 2), a release of furosemide in a buffer having pH 7.5 of at least 65% during a further consequtive hour (hour 3), and a release of furosemide in a buffer having pH 7.5 of at least 90% during a consequtive hour (hour 4).

FIG

## Description

<div align="center">DIURETIC COMPOSITION.</div>

Technical field

The present invention relates to a diuretic composition in multiple-unit dosage form and comprising furosemide, as the active ingredient.

The object of the present invention is to obtain a diuretic composition which when used shows a minimum of fluctuations of the plasma values, and to minimize side effects dependent on high plasma peak concentrations.

Background of the invention.

It is previously known a diuretic composition on the market, and from US-A-4,324,779, which composition shows an increased solubility, and thereby absorption in the body at a pH of about 4. This means that a release can take place already in the stomach, or very early in the upper part of the intestinal tract, which leads to very high plasma peaks. As pH varies in the stomach the release rate of furosemide will vary considerably. This means a varying load on the body, which is not suitable from a therapeutic point of view.

Furosemide is preferably used in long term treatment of cardial oedema, or oedema of light to medium severe degree, as well as in the treatment of light to medium severe hypertonia, particularly at diminished kidney function, or diabetes. Furosemide is also used at the indication congestive heart failure.

A desire for a composition having a more even release profile has thus been raised.

Description of the present invention

It has now been shown possible to be able to obtain a composition which meets the above given requirements by means of the present invention which is characterized in that the composition has a release in an aqueous medium having a pH of 1.5 of less than 4%, preferably less than 2% of furosemide after 1 hr (hour 1), a release in an aqueous medium having pH 5.5 of less than or equal to 7.5% of furosemide during a following hour (hour 2), a release in an aqueous medium having pH 7.5 of at least 65% during a further following hour (hour 3), and in the same medium having pH 7.5 of at least 90% of furosemide during a further hour (hour 4).

Further characteristics are evident from the accompanying claims.

By the present invention substantially no release of the active compound takes place in the stomach but the stomach can function as a depot of a multiple-unit dosage and it can by degrees continously give the composition in the form of pellets, diameter 0.5-1.5 mm, to the intestine, where these are dissloved and release furosemide with the desired rate in the higher pH existing therein.

By means of the invention the pH variations of the stomach, pH 1-5, are stopped from getting a real effect on the release, which otherwise will take place due to the low $pk_a$ of the furosemide, $pk_a = 3.9$.

According to US-A-4,324,779 it has not been possible to obtain a release below 10% at pH 5.5 during the second hour of release. This is also the reason why so large fluctuations have been obtained using such compositions. As a substantial release in pH 5.5 by such a type of compositions causes that a still higher release rate is obtained in a pH around 4.

The above given release rates of the compositions according to the present invention have been determined according to Dissotest (Sotax AG), flow 100 ml/hr, whereby the aqueous medium having pH 1.5 is a buffer solution of a mixture of 33.8 parts by volume of an aqueous solution containing 7.507 g of amino acetic acid, and 5.84 g of sodium chloride per litre, and 66.2 parts by volume of 0.1 N hydrochloric acid. The mix ture contains 0.01% polyoxyethylene sorbitan oleate.

The aqueous medium having pH 5.5 consists of a mixture of 3.9 parts of a 1/15 M disodium hydrogenphosphate solution, and 96.1 parts of a 1/15 M potassium dihydrogenphosphate solution, to which total mixture 0.01% polyoxyethylene sorbitan oleate has been added.

The aqueous medium having a pH of 7.5 consists of 6.805 g of potassium dihydrogenphosphate, and 1.630 g of sodium hydroxide per litre.

The release rate is determined at 37°C and normal pressure.

In a further test using an aqueous medium having pH 4.6 solely, the release of furosemide was less than 2% after one hour, and less than 4% after two hours in said medium when tested according to USP XXI <711> apparatus 2, 100 rpm. The aqueous medium consisted of an acetate buffer having the composition 30.69 g of acetic acid (99.8%), and 40.19 g of sodium acetate dissolved in destilled water to 10 litres. The present composition according to Example 1 below thus show an extreme low release of furosemide even at a pH above the $pk_a$ of the furosemide. It shall be understood that the test in pH 4.6 was carried out without any pre-exposure in pH 1.5.

The particle size of the release tested products has been 0.5 to 1.5 mm.

The invention will be described more in detail with reference to the examples given below, however, without being restricted thereto.

EXAMPLE 1.

A core comprising 30% furosemide, N-(2-furfuryl)-4-chloro-5-sulfamoyl-antranilic acid, was prepared in the following way. Microcrystalline cellulose, 4%, furosemide, and lactose, or mannitol and lactose, 40%, and starch, 20%, were mixed dry.

To the dry mixture a buffer solution of potassium dihydrogenphosphate and sodium hydroxide having a pH of 6.8 was added to the formation of a moist powder mass. The buffer solution is hereby a granulation liquid, which has a capacity to solve a certain part of the furosemide which gives a suitable binding of the mass. About 5% of the ingoing furosemide is calculated to become dissolved in said buffer solution. The powder mass is extruded in an extruder (NICASYSTEM), whereupon the extrudate is spheronized in a spheronizer (NICASYSTEM). The spherical granules obtained, diameter 0.5-1.5 mm, were then coated with an inner layer of ethyl cellulose, about 2%, and hydroxypropylmethyl cellulose, (Pharmacoat), about 2%, (40:60-60:40), and an outer layer consisting of hydroxypropyl methyl cellulose phtalate, $pk_a$ 5.5, in an amount of 1-10 % by weight, preferably 2-6% by weight.

The inner layer of ethyl cellulose and hydroxypropyl methyl cellulose can be 1.5-10% by weight. The hydroxypropyl methyl cellulose can be exchanged with hydroxypropyl cellulose, (Klucel), polyethylene glycol, (PEG), or a water soluble polyvinyl pyrrolidone in suitable amounts as well.

The hydroxypropyl methyl cellulose phtalate (HP 55) can be completely or partly exchanged with other anionic polymers as well, such as cellulose acetate phtalate, (CAP), or EUDRAGITE[R] L or EUDRAGITE[R] S types, which are copolymers.

The core above can contain 20 to 80% of furosemide, whereby 30 to 40% by weight are preferred.

The coating of the cores can either take place in a fluid bed or using a conventional pan coating, or using a so called Kugelcoater.

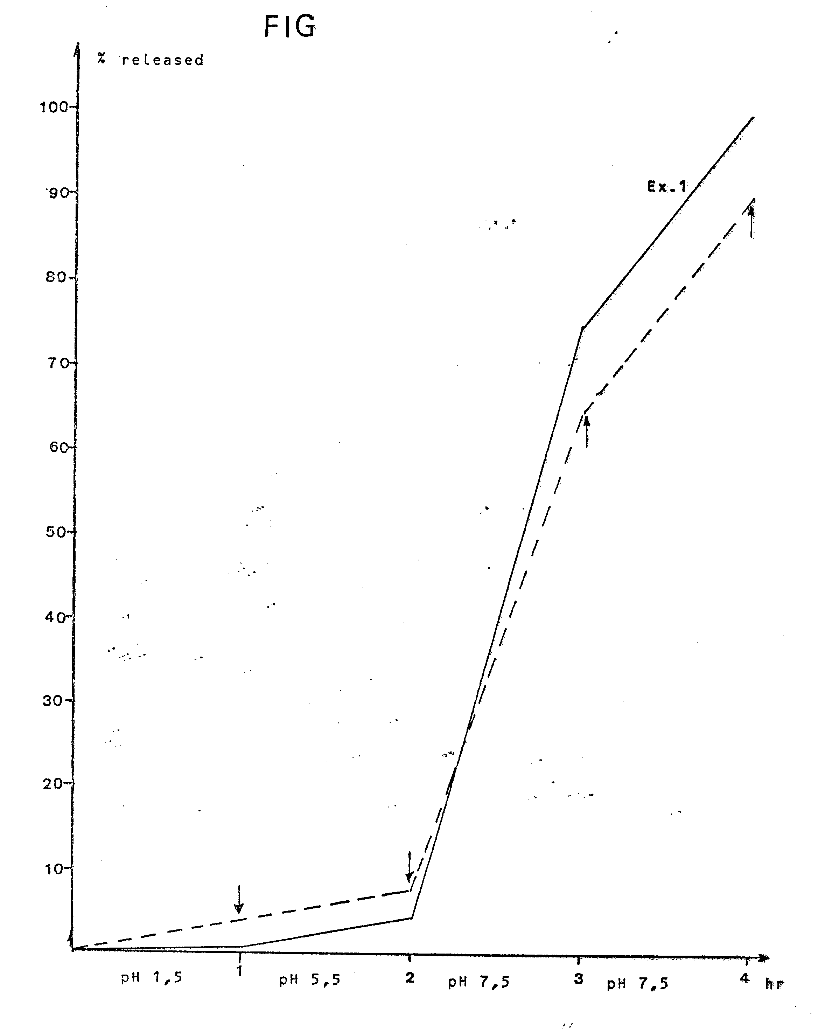

The coated core according to Example 1 above was tested in a Dissotest using the above given buffer solutions as release media. The result of this release test is evident from the attached FIG. 1, where the release curve is evident, as well as the release profile defined above.

EUDRAGITE L and S are copolymers of methacrylic acid and methacrylic acid methylester, whereby the relation acid to ester in the L-type is 1:1, and the relation acid to ester in the S-type is 1:2.

## Claims

1. A multiple-unit dose composition comprising furosemide, N-(2-furfuryl)-4-chloro-5-sulfamoyl-antranilic acid, as pharmaceutically active compound, characterized in that the composition in a release test using Dissotest, 100 ml/hr, in an aqueous medium having pH 1.5 gives a release of furosemide of less than 4% during 1 hr, and in the same test in an aqueous medium having pH 5.5 gives a release of furosemide of less than or equal to 7.5% during a following hour, (hour 2), and in the same test, in an aqueous medium having pH 7.5 gives a release of furosemide of at least 65% during a further following hour, (hour 3), and in the same test in the same aqueous medium having pH 7.5 gives a release of furosemide of at least 90% during a further following hour, (hour 4).

2. Composition according to claim 1, characterized in that the release in an aqueous medium having pH 1.5 is at most 2% during one hour.

3. Composition according to claim 1, characterized in that it comprises a core having a content of furosemide of 20 to 80% by weight, coated with a laminate having an inner layer of ethyl cellulose and hydroxypropyl methyl cellulose in an amount of at least 1.5% by weight, and an outer layer of hydroxypropyl methyl cellulose phtalate having $pk_a = 5.5$ in an amount of at least 1% by weight.

4. Composition according to claim 3, characterized in that the inner layer consists of ethyl cellulose and hydroxypropyl cellulose.

5. Composition according to claim 3, characterized in that the inner layer consists of ethyl cellulose and polyethylene glycol.

6. Composition according to claim 3, characterized in that the inner layer consists of ethyl cellulose and water soluble polyvinyl pyrrolidone.

7. Composition according to claim 3, characterized in that the outer layer consists of cellulose acetate phtalate.

8. Composition according to claim 3, characterized in that the outer layer consists of EUDRAGITE[R] L and/or EUDRAGITE[R]S copolymers.

9. Composition according to claim 3, characterized in that the inner layer comprises up to 10 % by weight.

10. Composition according to claim 3, characterized in that the outer layer comprises up to 10 % by weight.

FIG

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | EP-A-0 239 361 (KINAFORM TECHNOLOGY) * Pages 10,11, example 4; claims 1-5,9-14 * | 1,2 | A 61 K 31/635 A 61 K 9/54 |
| P,Y |  | 3-10 |  |
| X | EP-A-0 148 811 (LEJUS MEDICAL AB) * Page 1, lines 1-11; page 2, line 10 - page 5, line 10; claims 1-3,5-7,10 * | 1-10 |  |
| A | EP-A-0 080 341 (A/S ALFRED BENZON) * Pages 31,32, example 6 * |  |  |
| Y | GB-A-2 025 227 (BOEHRINGER INGELHEIM) * Page 1, lines 74-98; page 2, lines 48-60,120-125; page 8, lines 31-38 * | 3-10 |  |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 19-04-1988 | BENZ K.F. |